# EUROPEAN PATENT APPLICATION

(11) **EP 0 609 879 A1**
(43) Date of publication of application: **10.08.1994**
(21) Application number: 94101644.6
(22) Date of filing: 03.02.1994
(51) Int. Cl.: D21H 17/16

(54) **Sizing agent for papermaking**

(30) Priority: 03.02.1993 JP 37344/93; 22.02.1993 JP 54695/93
(71) Applicant: Mitsubishi Oil Company, Limited, Minato-ku Tokyo (JP)
(72) Inventor: Hatanaka, Shigeto, Yokohama-shi, Kanagawa (JP); Kouchi, Takeshi, Yokohama-shi, Kanagawa (JP)
(74) Representative: Hansen, Bernd, Dr. Dipl.-Chem.

(57) **Abstract**

A sizing agent for papermaking with which the generation of stain on paper is controlled due to the decreased generation of stain at a drier site and which shows excellent sizing effect. The sizing agent contains the addition reaction product of linear internal olefins having a carbon atom distribution in which those having 17 or less carbon atoms comprise less than 5 weight % and those having 18 or more carbon atoms comprise 95 weight % or more, and maleic anhydride.

## Description

### FIELD OF THE INVENTION

The present invention relates to an alkenylsuccinic anhydride type sizing agent for papermaking which is emulsified for use and to a sizing agent with which the generation of a stain on paper is suppressed because of decreased generation of a stain at a drier site and which shows excellent sizing effect.

### BACKGROUND OF THE INVENTION

Generally in manufacturing paper, a lot of filler such as talc and clay is used for improvement in printing aptitude and whiteness degree and for the provision of opaqueness. In recent years, the increasing trend has been to replace this with calcium carbonate which is abundantly present and can be obtained at an inexpensive price.

However, since a so-called anionic type sizing agent such as a rosin series sizing agent has conventionally been used as the sizing agent for papermaking in an acidic sizing process in which the sizing agent is fixed on pulp with aluminum sulfate, the use of calcium carbonate as a filler generates the defect that calcium carbonate is decomposed by the acidity in a papermaking system.

Accordingly, in order to solve this problem, various neutral sizing agents have been proposed by which sizing can be carried out at a neutral region or an alkaline region without using aluminum sulfate.

For instance, U.S. Patent 3,102,064 proposes a neutral sizing agent represented by the following formula: 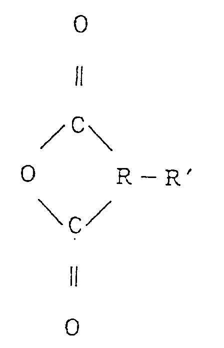
wherein R represents a dimethylene or trimethylene radical and R' is a hydrophobic group containing more than five carbon atoms which may be selected from the group consisting of alkyl, alkenyl, aralkyl or aralkenyl groups. U.S. Patent 3,821,069 proposes a sizing agent comprising the reaction product of maleic anhydride with an internal olefin, which is represented by the following formula:

Rₓ-CH₂-CH=CH-CH₂-R_{y}

wherein Rₓ and R_{y} each represent an alkyl radical containing at least four carbon atoms.

Further, JP-A-57-154495 (the term "JP-A" as used herein means an unexamined published Japanese patent application) proposes a sizing agent comprising a mixed alkenylsuccinic anhydride obtained by adding maleic anhydride to the mixture of linear internal olefins which have from 8 to 18 carbon atoms and in which the double bonds are almost evenly distributed at the respective positions excluding an α-position. JP-A-59-179898 proposes a sizing agent containing the reaction product obtained by the addition reaction of a linear type olefin mixture in which the proportion of olefins having from 14 to 36 carbon atoms and having double bonds at the 2-position, the 3-position and the 4-position, reside in 10 to 65 mole %, respectively, and the total amount thereof is 70 mole % or more, and maleic anhydride, and/or the hydrogenated product of the above reaction product. Further, as described in JP-A-60-99099, emulsifiers are investigated as well.

These alkenylsuccinic anhydride type sizing agents for papermaking are usually emulsified with a homomixer and a homogenizer using a water soluble high molecular compound such as cationized starch and a surface active agent such as polyoxyalkylene aryl ether, and are added to paper in the form of an aqueous emulsion. In any cases, because of insufficient emulsification and stability after the emulsification, a satisfactory sizing effect could not be obtained, and there was involved the problem that a stain was generated in a papermaking system at the part extending from a chest to a press, which is called a wet end. As described in U.S. Patent 5,246,491, there is available for this problem, a method in which there are utilized for controlling the particle size of an emulsion, a 1 : 2 adduct by-produced by reacting an olefin and maleic anhydride to manufacture alkenylsuccinic anhydride (a 1 : 1 adduct) and the decarboxylated product of the 1 : 2 adduct to thereby reduce stain.

### SUMMARY OF THE INVENTION

As described above, these alkenylsuccinic anhydride type sizing agents have been improved in terms of sizing effect and decrease in a stain at a wet end. However, the generation of stain at a drier part has not yet been reduced and a spot on paper which is caused by the stain put on the paper has never been solved. The research in the past has not clarified the generating mechanism of this spot on paper. In the case where the spot on paper is generated, the part thereof must be removed in a rolling operation for a finished product, and the reduction of the spot on paper has become an important problem as well from the viewpoint of productivity. However, it is difficult to solve this problem using conventional techniques such as the structure of raw material olefin, the kind of an emulsifier, and the improvement in an emulsifying method, and therefore sizing agents obtained on an entirely new standpoint are desired.

An object of the present invention is to provide a sizing agent having less generation of stain on paper because of the lesser generation of stain at a drier part and showing an excellent sizing effect.

Diligent investigations made by the present inventors in order to achieve this object have resulted in finding the cause of generation of stain at a drier part, and the finding that the stain generated at the drier part is the cause for the generation of the stain on paper and has led to the present invention. That is, the present inventors have noticed the stain at the drier part and investigated it. The results thereof show that the stain comprises alkenylsuccinic acid generated by the reaction of alkenylsuccinic anhydride with water and a calcium alkenylsuccinate salt generated by the reaction thereof with calcium carbonate. This means that alkenylsuccinic anhydride has evaporated at the drier part. The boiling point of this alkenylsuccinic anhydride is 400°C or higher at normal pressure and it was unexpected that it had evaporated at the drier part with the temperature being not much more than 150 °C. It is presumed that alkenylsuccinic anhydride stands in a steam distillation condition by water vaporized from a wet paper and is distilled under a reduced pressure for evaporation. It is considered that the evaporated alkenyl-succinic anhydride sticks to each part of the drier and a drier canvas and soon reacts with water and calcium carbonate used as a filler to become alkenylsuccinic acid and the calcium salt thereof, whereby stain is formed. It is assumed that this stain peels and falls on the surface of the paper to generate a stain on the paper.

To be further surprising, while C16 was 57 weight % and C18 was 43 weight % in carbon atom distribution in the raw material olefin for the sizing agent used, C16 was far condensed according to the analytical result of the raw material olefin in the alkenylsuccinic acid and in the calcium salt thereof obtained as the stain at the drier part. The analytical result is shown in Table 1.

**Table 1**

| | | |
|---|---|---|
| Fraction: | Acetone soluble | Acetone insoluble |
| Component: | Hydrized ASA | ASA calcium salt and others |
| Content: | 10 wt% | 90 wt% |
| Raw material carbon atom distribution: | | |
| C16 | 76 wt% | 75 wt% |
| C18 | 24 wt% | 25 wt% |
| ASA: alkenylsuccinic anhydride Raw material carbon atom distribution in ASA used: C16: 57 wt% and C18: 43 wt% | | |

This allowed the volatility of alkenylsuccinic anhydride to be noticed and this was diligently researched. As a result thereof, it has been discovered that the increase in the number of carbon atoms in the raw material olefin in alkenylsuccinic anhydride could reduce the evaporated amount of alkenylsuccinic anhydride and prevent stain at a drier part and resultant spot on the paper, which has led to the present invention. As described above, it has thus far not been an object to control the number of carbon atoms in the raw material olefin in an alkenylsuccinic anhydride type sizing agent in order to prevent stain at the drier part and resultant spot on the paper. Thus the present invention provides an alkenylsuccinic anhydride type sizing agent which has been discovered based on an entirely new standpoint. As a matter of fact, almost all of the alkenylsuccinic anhydride type sizing agents commercially available on the market at present are manufactured from a raw material containing a lot of olefins with the 17 or less carbon atoms.

### BRIEF DESCRIPTION OF THE DRAWINGS

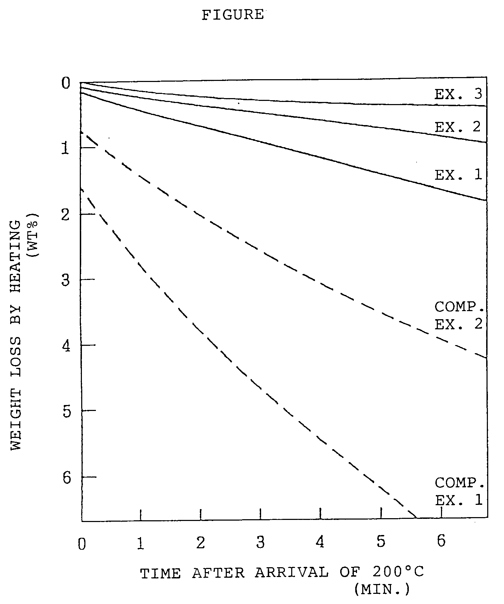

The figure shows the heating loss measurement results of the olefin maleic anhydride addition reaction products obtained in Examples 1 to 3 and Comparative Examples 1 to 2 with a thermo-balance.

### DETAILED DESCRIPTION OF THE INVENTION

To describe the present invention in more detail, the present invention provides a sizing agent for papermaking containing as an effective component the addition reaction product of a linear internal olefin having a carbon atom distribution in which those linear internal olefins having 17 or less carbon atoms comprise less than 5 weight % and those linear internal olefins having 18 or more carbon atoms comprise 95 weight % or more (based on the total amount of linear internal olefins to obtain the addition reaction product), and maleic anhydride, more preferably the sizing agent for papermaking containing the addition reaction product of a linear internal olefin having a carbon atom distribution in which those linear internal olefins having 17 or less carbon atoms comprise less than 1 weight %, those linear internal olefins having the 18 to 20 carbon atoms comprise 69 to 100 weight % and those linear internal olefins having 21 or more carbon atoms comprise less than 30 weight % (based on the total amount of linear internal olefins used to obtain the addition reaction product), and maleic anhydride.

There can be used as the linear internal olefin which is the raw material, those obtained by isomerizing a linear α-olefin having the above carbon atom distribution with a catalyst and those obtained by dehydrogenating an n-paraffin. In order to carry out a separation by distillation in order of the number of carbon atoms with good precision, it is desirable to use isomerized ethylene oligomer α-olefins manufactured by the polymerization of ethylene and having a carbon atom number with an interval of 2. The α-olefin can be isomerized according to the method described, for example, in JP-A-61-119797.

In order to maximize the performances of the present invention, a higher proportion of olefin with 18 to 20 carbon atoms is more preferred. The increase in the content of olefin having 17 or less carbon atoms leads to an increase in the generation of drier stain, and therefore a content of an olefin having a 17 carbon atoms of less than 1 weight % is preferred but a content of less than 5 weight % can provide a reduction in the drier stain as compared with a conventional sizing agent. An olefin with 21 or more carbon atoms may be contained. However, it is preferably controlled to less than 30 weight % since not only the increase in the content thereof increases a fluid point to make the handling thereof difficult but also temperature rises in emulsifying result and the diameter of an emulsified particle becomes coarse, whereby sizing effect is reduced. Further, those containing a small amount of branched olefin, α-olefin and paraffin also can be used as raw material olefin.

Any conventional processes may be used for the addition reaction of linear internal olefin and maleic anhydride, and a catalyst may be or may not be used. As an example, the temperature is raised to 180 to 250°C at normal pressure or under pressure, preferably under an inert atmosphere of nitrogen gas in the absence of a catalyst, and the reaction is carried out at that temperature for 1 to 30 hours. The loaded ratio of both reactants is not required to be specifically limited. They are used preferably in a ratio of 0.5 to 3 moles of maleic anhydride to 1 mole of olefin. Unreacted olefin and maleic anhydride are removed from the system by distillation.

The sizing of pulp with the sizing agent for papermaking containing the addition reaction product thus obtained of linear internal olefin and maleic anhydride is usually carried out by adding to paper in the form of an emulsion obtained by uniformly dispersing this sizing agent in water.

In order to improve the dispersion in water, various emulsifiers can be used singly or in combination, including, for example, polyoxyalkylenesorbitan fatty acid ester, polyoxyalkylenesorbitol fatty acid ester, polyoxyalkylenealkyl ether, polyoxyalkylenealkyl aryl ether, fatty acid ester of polyhydric alcohol, a sulfuric acid ester salt of polyoxyalkylenealkyl ether, polyoxyalkylenealkyl ether phosphoric acid ester, polyoxyalkylenealkyl aryl ether phosphoric acid ester, and polyoxyalkylenearalkyl aryl ether phosphoric acid ester, and various cationic compounds as a suspending agent, for example, cationized starch and cationic polyacrylamide.

The amount of the emulsifier used in combination with the maleic anhydride addition reaction product of the present invention is determined considering the kind of emulsifying equipment and the dispersibility. Usually, it is preferably 0.5 to 20 weight %, more preferably 1 to 10 weight % based on the addition reaction product of linear internal olefin and maleic anhydride.

Usually, the amount of a cationic compound which is used as a suspending agent is preferably 30 to 600 weight %, more preferably 100 to 300 weight % based on the addition reaction product of linear internal olefin and maleic anhydride.

As the emulsifying equipment, the equipment used for emulsifying a conventional alkenylsuccinic acid type sizing agent, such as a homomixer, a homogenizer, a nozzle type emulsifying equipment, an orifice type emulsifying equipment, or a turbine type emulsifying equipment, can be used.

After emulsifying, the sizing agent of the present invention can be added to a pulp slurry at an arbitrary stage in the papermaking process.

The amount of the sizing agent of the present invention is changed according to the kind of pulp used, the papermaking condition and the application of paper. Usually, it is used preferably in 0.05 to 3.0 weight % based on the dry weight of the pulp.

In order to fix the sizing agent of the present invention on pulp, cationic compounds such as a cationized starch, a cationic polyacrylamide, and a polyaminepolyamide-epichlorohydrin resin are usually used as the fixing agent. The amount used is preferably 0.01 to 5.0 weight %, more preferably 0.03 to 3.0 weight % based on the dry weight of the pulp. Any of these fixing agents can be added simultaneously with, before or after the addition of the sizing agent. However, it is added preferably after the addition of the sizing agent in order to obtain an optimum fixing effect.

The sizing agent of the present invention can be used in combination with various sizing agents out of the present invention in an arbitrary proportion, if desired.

Furthermore, in sizing, various kinds of fillers such as talc, clay, titanium oxide, calcium carbonate, calcium sulfate, and aluminum hydroxide, can be used as filler for paper.

To further illustrate the present invention in greater detail, the following non-limiting examples are provided below. Unless otherwise indicated herein, all parts, percents, ratios and the like are by weight.

### EXAMPLE 1

Ethylene oligomer α-olefin containing C₁₈ (C₁₆: 0.1 weight %, C₁₈: 99.9 weight %) as a main component was isomerized with a nickel octanoic acid catalyst and an alkyl-aluminum catalyst. Maleic anhydride (583 g) (mole ratio to olefin: 1.5) was added to the linear internal olefin thus obtained (double bond position distribution: 1-position: 0 mole %, 2-position: 13 mole %, 3-position: 12 mole %, 4-position: 15 mole %, and 5⁺-position: 60 mole %) (1000 g), and the reaction was carried out in an autoclave at 215°C for 8 hours in the absence of a catalyst. Unreacted olefin and maleic anhydride were removed from the reaction solution by distillation under reduced pressure, whereby 1320 g of the olefin maleic anhydride addition reaction product was obtained.

### EXAMPLE 2

Ethylene oligomer α-olefin containing C₂₀ (C₁₈: 0.4 weight %, C₂₀: 98.2 weight %, C₂₂: 1.4 weight %) as a main component was isomerized in the same manner as that in Example 1. Maleic anhydride (525 g) (mole ratio to olefin: 1.5) was added to the linear internal olefin thus obtained (double bond position distribution: 1-position: 0 mole %, 2-position: 12 mole %, 3-position: 11 mole %, 4-position: 14 mole %, and 5⁺-position: 63 mole %) (1000 g), and the olefin maleic anhydride addition reaction product (1250 g) was obtained according to the operating process in Example 1.

### EXAMPLE 3

Ethylene oligomer α-olefin with a carbon atom distribution (C₁₈: 0.5 weight %, C₂₀: 28.0 weight %, C₂₂: 25.0 weight %, C₂₄: 19.0 weight %, C₂₆: 15.0 weight %, C₂₈: 9.0 weight %, C₃₀ or more: 3.5 weight %) was isomerized in the same manner as that in Example 1. Maleic anhydride (470 g) (mole ratio to olefin: 1.5) was added to the linear internal olefin thus obtained (double bond position distribution: 1-position: 0 mole %, 2-position: 9 mole %, 3-position: 9 mole %, 4-position: 12 mole %, and 5⁺-position: 70 mole %) (1000 g), and the olefin maleic anhydride addition reaction product (1185 g) was obtained according to the operating process in Example 1. This product had a melting point of 30°C and was a solid at ordinary temperature.

### COMPARATIVE EXAMPLE 1

Maleic anhydride (655 g) (mole ratio to olefin: 1.5) was added to linear internal olefin (double bond position distribution: 1-position: 0 mole %, 2-position: 13 mole %, 3-position: 12 mole %, 4-position: 17 mole %, and 5⁺-position: 58 mole %) (1000 g) obtained by isomerizing ethylene oligomer α-olefin with a carbon atom distribution (C₁₆: 99.8 weight %, C₁₈: 0.2 weight %) in the same manner as that in Example 1, and the olefin maleic anhydride addition reaction product (1390 g) was obtained according to the operating process in Example 1.

### COMPARATIVE EXAMPLE 2

Ethylene oligomer α-olefin with a carbon atom distribution (C₁₆: 56.8 weight %, C₁₈: 43.2 weight %) was isomerized in the same manner as that in Example 1. Maleic anhydride (623 g) (mole ratio to olefin: 1.5) was added to linear internal olefin thus obtained (double bond position distribution: 1-position: 0 mole %, 2-position: 12 mole %, 3-position: 12 mole %, 4-position: 15 mole %, and 5⁺-position: 61 mole %) (1000 g), and the olefin maleic anhydride addition reaction product (1360 g) was obtained according to the operating process in Example 1.

The olefin maleic anhydride addition reaction products obtained in Examples 1 to 3 and Comparative Examples 1 to 2 were subjected to a heating loss measurement and a sizing effect measurement test.

### Heating Loss Measurement:

Five mg of each of the olefin maleic anhydride addition reaction products obtained in Examples 1 to 3 and Comparative Examples 1 to 2 was put on a thermo-balance (TGA-50 manufactured by Shimadzu Corp.) and heated to 200°C at 10°C/minute in a nitrogen flow with the flow velocity of 25 ml/minute. Then, a weight loss rate at 200°C was measured. The results are shown in the figure. It can be seen that the sizing agents of the present invention obtained in Examples 1 to 3, which contain only less than 5 weight % of C₁₇ in raw material olefin have very small volatile losses as compared with those obtained in Comparative Examples 1 to 2, in which a lot of olefin with C₁₇ or lower was present.

The samples in Example 1 and Comparative Example 1 were subjected to a papermaking test in a commercial machine to find that the degrees of a drier stain and the stain of a spot on paper were markedly excellent in Example 1.

### Sizing Effect Measuring Test:

Polyoxyethylene nonylphenyl ether (HLB 13) (0.5 g) was added as an emulsifier to the olefin maleic anhydride addition reaction product (10 g) and mixed well. A 1.5 % cationized starch solution (99 g) was added to this mixture (1 g) and an emulsification was carried out with the TK homomixer model M manufactured by Tokushukika Ind. Co., Ltd. at 7000 r.p.m. for 2 minutes.

The emulsion thus prepared was added to a pulp slurry (L.B.KP, CSF=430 ml) so that the ratio of the olefin maleic anhydride addition reaction product became 0.1, 0.15 or 0.2 weight % (based on a pulp solid component), and 0.8 weight % of cationized starch (based on the pulp solid component) and 0.03 weight % of cationic polyacrylamide (based on the pulp solid component) were added under stirring, followed by carrying out papermaking with a TAPPI standard machine according to an ordinary process. The filler used was a light calcium carbonate and the amount used was 20 weight % (based on the pulp solid component).

Next, the wet paper thus obtained was dehydrated by pressing and dried in a rotary drier by heating at 105°C for 1 minute, followed by subjecting it to a humidity adjustment in a room of 20°C and the relative humidity of 65 %. The weight of the handmade paper obtained after the humidity adjustment corresponded to 60 g/m². The sizing degree of this handmade paper was measured by a JIS P 8122 Stöckigt sizing degree test method.

The results are shown in Table 2. Example 3 in which a lot of C₂₁ or higher was contained in raw material olefin showed a little lower sizing effect but the other sizing agents had almost the same effect.

**Table 2**

| (Sizing Effect Evaluation Results) | | | |
|---|---|---|---|
| | Stöckigt Sizing Degree (sec.) | | |
| Sizing Agent Addition Rate: | 0.1 wt% | 0.15 wt% | 0.2 wt% |

| Sizing agent: | | | |
|---|---|---|---|
| Example 1 | 7 | 18 | 27 |
| Example 2 | 6 | 17 | 25 |
| Example 3 | 4 | 15 | 21 |
| Comparative Example 1 | 6 | 17 | 28 |
| Comparative Example 2 | 7 | 18 | 26 |

While the invention has been described in detail and with reference to specific embodiments thereof, it will be apparent to one skill in the art that various changes and modifications can be made therein without departing from the spirit and scope thereof.

## Claims

1. A sizing agent for papermaking containing an addition reaction product of linear internal olefins having a carbon atom distribution in which those olefins having a 17 or less carbon atoms comprise less than 5 weight % of the total olefin weight and those olefins having 18 or more carbon atoms comprise 95 weight % or more of the total olefin weight, and maleic anhydride.

2. The sizing agent for papermaking as claimed in claim 1, containing the addition reaction product of a linear internal olefin having a carbon atom distribution in which those olefins having 17 or less carbon atoms comprise less than 1 weight %, those olefins having a 18 to 20 carbon atoms comprise 69 to 100 weight % and those olefins having a 21 or more carbon atoms comprise less than 30 weight %, and maleic anhydride.
